(19) Europäisches Patentamt
European Patent Office
Office européen des brevets

(11) Publication number: **0 148 795 B1**

# (12) EUROPEAN PATENT SPECIFICATION

(45) Date of publication of patent specification: **18.04.90**

(51) Int. Cl.[5]: **C 07 D 277/10,** A 01 N 25/32, C 07 D 263/10

(21) Application number: **85400009.8**

(22) Date of filing: **04.01.85**

## (54) Compounds exerting an antidotal activity for the defence of agrarian cultivations against the toxic action of non-selective herbicides.

(30) Priority: **06.01.84 IT 1904784**

(43) Date of publication of application: **17.07.85 Bulletin 85/29**

(45) Publication of the grant of the patent: **18.04.90 Bulletin 90/16**

(84) Designated Contracting States: **BE CH DE FR GB LI NL**

(56) References cited:
**EP-A-0 027 019**
**EP-A-0 044 201**
**DE-A-1 545 720**
**FR-A-1 506 154**
**FR-A-2 150 841**
**US-A-3 293 245**
**US-A-3 564 004**

**JOURNAL OF THE CHEMICAL SOCIETY, serie C, 1967, pages 20-25, London, GB; G. HOLAN et al.: "2-Trihalogenomethylbenzazoles. Part I. Formation"**

(73) Proprietor: **Montedison S.p.A.**
**31, Foro Buonaparte**
**I-20121 Milan (IT)**

(72) Inventor: **Palla, Ottorino**
**5 Via Rampazzini**
**Crema (Cremona) (IT)**
Inventor: **Camaggi, Giovanni**
**32 Via Defendente**
**Lodi (Milan) (IT)**
Inventor: **Gozzo, Franco**
**52/P, Via Triulziana**
**San Donato Milanese (Milan) (IT)**
Inventor: **Menconi, Augusto**
**1,Via A Grandi**
**Crema (Cermona) (IT)**
Inventor: **Signorini, Ernesto**
**51 Via Matteotti**
**Malnate (Varese) (IT)**

(74) Representative: **Hirsch, Marc-Roger**
**Cabinet Hirsch 34 rue de Bassano**
**F-75008 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 148 795 B1

**Description**

This invention relates to a new method of defending useful plants against the phytotoxic effects of herbicides. Furthermore, the invention relates to compositions for reducing the damage caused by non-selective herbicides towards useful cultivations and also concerns derivatives of 2-thiazoline and of 2-oxazoline.

It is known that the herbicides belonging to the classes of chloroacetanilides, thiolcarbamates, triazines, etc. are useful compounds in the fight against the infesting plants of important agrarian cultivations.

However, many such herbicides also exert their toxic action towards certain useful cultivations, such as, for example, maize and sorghum, and by consequence, since they are non-selective, they cannot be used for killing weeds infesting such cultivations.

The availability of antidotes, i.e. of compounds which defend the useful cultivations against the action of the herbicides without simultaneously suppressing the herbicidal activity towards infesting plants, permits to use these herbicides also for protecting those useful cultivations which would otherwise be damaged.

Among the main herbicides which prove to be phytotoxic for certain useful cultivations may be cited those belonging to the class of the chloroacetanilides, which includes, for example, N-methoxymethyl-2,6-diethyl-chloroacetanilide (common name: Alachlor), N-butoxymethyl-2,6-diethyl-6-allyl-chloroacetanilide (item M8669), and those belonging to the class of the thiolcarbamates, which includes for exmaple N,N-diisopropyl-S-(2,3-dichloroallyl)-thiolcarbamate (common name Triallate); N,N-diethyl-S-(4-chlorobenzyl)-thiolcarbamate (common name Benthiocarb); N,N-dipropyl-S-ethyl-thiolcarbamate (common name Eptam).

Compounds are known belonging to various chemicals classes, which are capable of protecting useful cultivations against the toxic action of herbicides. For example, dichloroacetamides useful as antidotes have been described in US patent No. 4 021 224 (Stauffer) and in US patent No. 4 228 101 (Montedison).

The Applicant's research has now revealed a new method of defending useful plants against the phytotoxic effects of herbicides consisting in treating the plants or parts thereof or the soil in which said plants grow with antidotally effective amounts, ranging from 0,03 to 10 parts by weight of herbicide, of a compound of general formula (I):

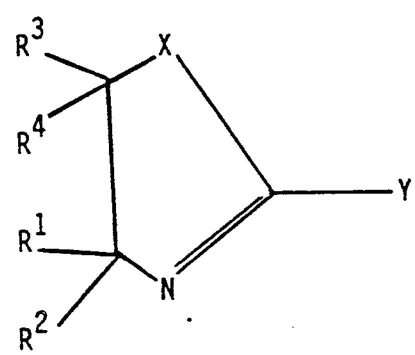

(I)

wherein:

X = S, O;

Y = alkyl $C_1$—$C_4$ substituted by one or more halogen atoms selected from the group consisting of Cl, Br, and F;

$R^1$, $R^2$, $R^3$ and $R^4$, similar to or different from one another, are hydrogen, or a $C_1$—$C_4$ alkyl.

The toxic action of non-selective herbicides, belonging, for example, to the class of chloroacetanilides and of thiolcarbamates, towards useful cultivations can be highly reduced or eliminated without simultaneously suppressing the herbicidal action towards infesting plants, if the compounds of formula I are utilized as antidotes.

Thus, a further object of the present invention consists in a method of reducing the damage towards useful plants caused by non-selective herbicides belonging, e.g. to the class of the chloroacetanilides or of the thiolcarbamates, such method consisting in treating the plants or the soil in which they grow with an effective amount of an antidote of formula I, either as such or as a suitable composition.

The invention also relates to the compounds of general formula (I) wherein:

X is an atom of S;

Y is an alkyl $C_1$—$C_4$ substituted by one or more halogen atoms selected from the group consisting of $C_1$, Br, F provided that Y is not $CCl_3$;

$R^1$, $R^2$, $R^3$ and $R^4$, similar to or different from one another are hydrogen or an alkyl $C_1$—$C_4$.

Another object of this invention is represented by compositions containing a compound of formula I as an active ingredient besides inert carriers and optionally herbicides and other additives, useful for treating

the seeds of useful plants, the plants themselves or the soil in which they grow.

A further object of the present invention are the seeds of useful plants when treated with an effective amount of a compound of formula I.

The compounds of formula I are prepared according to general methods adopted in the synthesis of 2-thiazolines. For example, it is possible to utilize the Ritter reaction between a nitrile and a diol or a 2-mercapto-alcohol (Organic Reactions, vol. 17 (1969), 237) according to the following equation:

1) 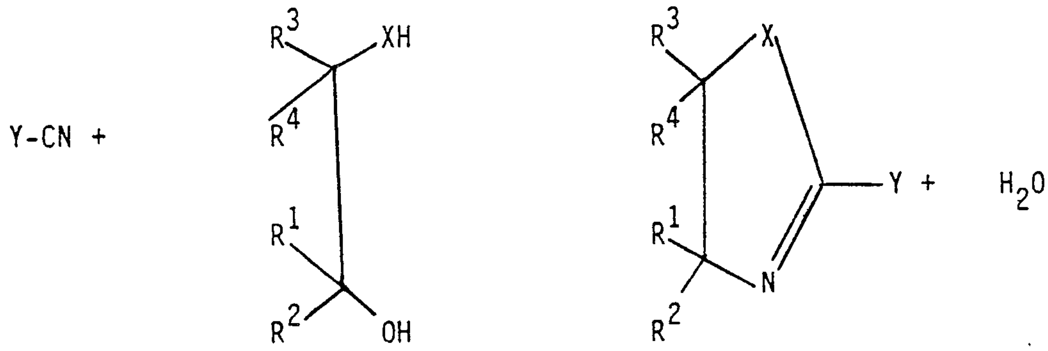

wherein X, Y, $R^1$, $R^2$, $R^3$ and $R^4$ are the same as defined hereinabove.

In particular, when X = 0, the compounds of formula (I) can also be obtained by reacting a haloid salt of a 2-halo-alkylamine with the proper anhydride (Gabriel, Bereichte, 22, 2221) according to the equation:

2) 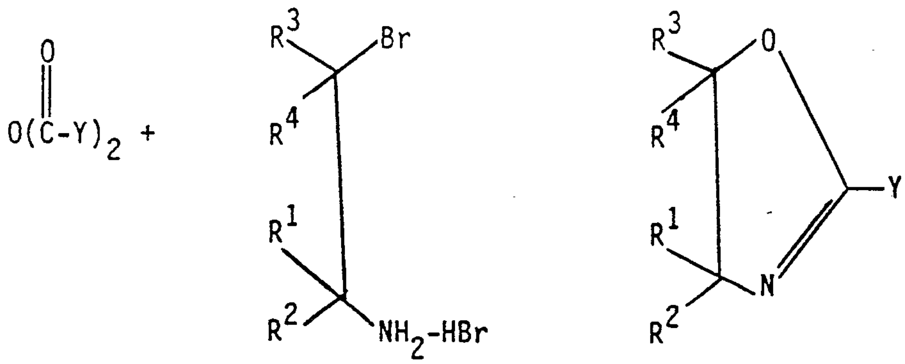

Furthermore, in particular, when X = S, the compounds of formula (I) can be obtained by reacting a disulphide of a 2-mercaptoalkylamine with the proper anhydride (Gabriel, Berichte, 24, 1117) according to the equation:

3) 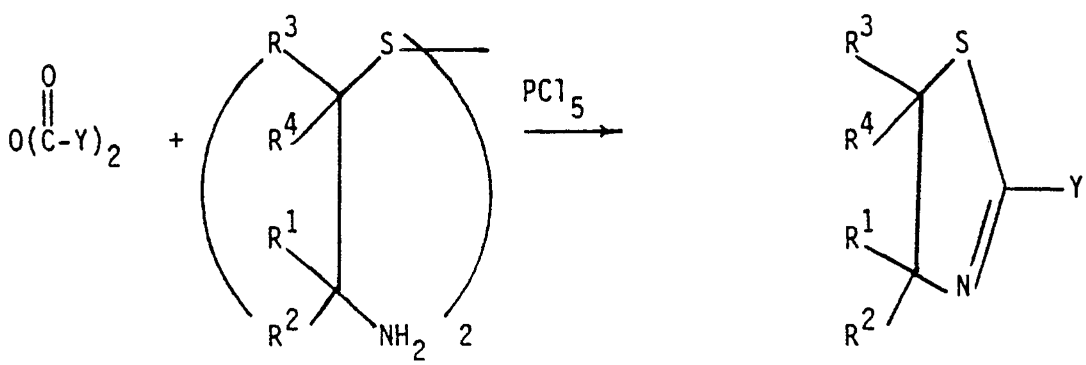


Similarly, another general method which is very useful for synthetizing the compounds (I) in which X = S, consists in treating a nitrile with a 2-mercaptoalkylamine according to the equation:

4) 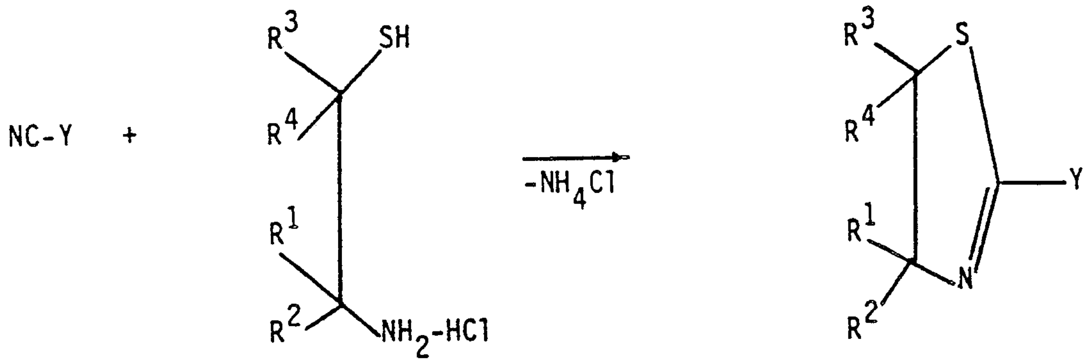


This reaction can be carried out in a suitable alcoholic solvent, for example methanol or ethanol, under reflux conditions.

As mentioned hereinbefore, the antidotes of formula (I) exert a remarkable antidotal action which is much higher than that of antidotes of the prior art, for example, much higher than the action of the antidote N,N-diallyldichloroacetamide (R-25788) described in the above-cited US patent No. 4 021 224 (comparative example No. 4).

The antidotes of formula (I) can be applied to the useful cultivations according to different methods.

For example, they can be utilized for a preventive dressing of the seeds, so that the plant which will develop from said seeds may be protected against the toxic action of non-selective herbicides. As an alternative, the compounds of formula (I) can be used for treating the plant itself or the soil in which it grows. In this case, the antidotes can be distributed either alone or preferably combined with the non-selective herbicide.

The various kinds of application require different conditions which influence the practical aspects of the treatment, such as antidote amount, treatment period and type of composition.

Other factors influencing the practical aspects of the treatment are the type of cultivation to be protected, the non-selective herbicide which is used, climatic and environmental conditions.

When the antidote is utilized in a preventive dressing of the seeds, it can be utilized as such or preferably in the form of a suitable composition.

The compositions for the dressing of the seeds may be in the form of powders, wettable powders or emulsifiable concentrates and generally consists of the active compounds in amounts ranging from 0.5 to 95% by weight and of the usual inert carriers which, depending on the type of composition, may be solid, such as talc, silica, diatomaceous earth, bentonite, alkyl-aromatic hydrocarbons, acetone, cyclohexanone and mixtures thereof.

Proper additives, such as surfactants, wetting agents, dispersants and mixtures thereof can also be present in the compositions.

A specific example of a composition in powder form for the seed dressing is the following:

| | |
|---|---|
| Compounds of formula (I) | 25—75% by weight |
| mixture of a wetting agent, a dispersant and an adhesion-promoting agent | 1—5% by weight |
| solid inert carrier | 20—74% by weight |

Some examples of useful wetting agents are polyoxy-ethylated nonyl-phenols, sodium-alkylnaphtalensulphonates, sodium alkylsulphosuccinates; some example of dispersants are lignosulphonates of sodium, calcium or aluminium, sodium alkylnaphtalensulphonates condensed with formaldehyde, maleic anhydride/diisobutylene copolymers; some examples of adhesion-promoting agents are glycols, glycerine, polygylcols, arabic gum, starch, sodium polymethacrylate with different molecular weights.

All these additives are well known in the formulative field and are commercially also available in already prepared mixtures.

The above-mentioned compositions are prepared by mixing the ingredients and by homogenizing them by means of grinding until the desired particle size is obtained.

Such compositions may be used as such for the dry seed-dressing, or may be diluted with water for wet seed-dressing.

As indicated hereinabove, the antidote amount to be distributed onto the seeds varies as a function of various factors; however, it is generally sufficient to use product amounts ranging from 0.1 to 100 g/kg of seed.

Of course, the treatments carried out directly on the plant or in the medium in which it grows require use of the antidote in the form of a suitable composition according to common practice for this kind of application.

In the applications in which the antidote is distributed onto the vegetation or in the soil along with the

non-selective herbicide in a single formulation, the type of formuklation and the content vary both in relation to the facts disclosed hereinbefore and in relation to the type of herbicide utilized and to the characteristics thereof.

The antidote amount to be utilized generally ranges from 0.03 to 10 parts by weight per 100 parts by weight of herbicide.

Among the cultivations which can be defended against the toxic action of non-selective herbicides due to the use of antidotes of formula (I), other than maize, may be cited beet, sorghum and wheat.

In order to better illustrate the present invention, the following examples are given.

Example 1

Preparation of 2-trichloromethyl-2-thiazoline (compound No. 1).

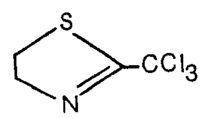

5.65 g (0.05 moles) of 2-mercaptoethylamine hydrochloride and 7.2 g (0.05 moles) of trichloroacetonitrile in 50 ml of methanol were heated at reflux during 3 hours. At the end of this step, the solvent was distilled off under reduced pressure and the residual oil was diluted with 100 ml of diethylether.

The ammonium chloride which had formed during the reaction was separated by filtration and the ethereal solution, after washing with water, drying with $Na_2SO_4$ and evaporation of the solvent, 9 g (0.44 moles) of the product were obtained, in the form of a liquid having a boiling point of 95—100°C at 0,02666 bars (20 mm of Hg) I.R.: $\gamma$ (C=N) 1615 $cm^{-1}$.

Example 2

Preparation of 2-dichloromethyl-2-thiazoline (M13302) (compound No. 2)

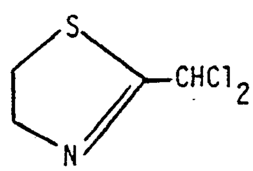

Starting from dichloroacetonitrile and by means of a process analogous to that described in example 1, the compound indicated in the title and having the following characteristics was prepared:

Physical state: pale-yellow oil,
I.R.: $\gamma$ (C=N) 1620 $cm^{-1}$.
1H-NMR ($CDCl_3$)

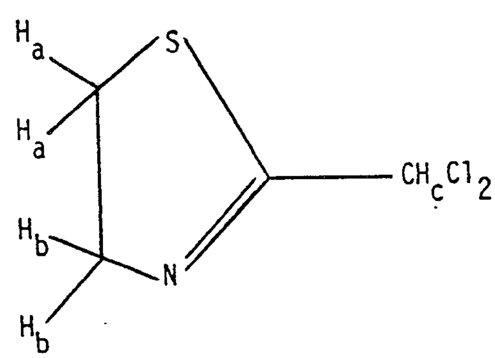

δ (ppm): $H_a$ 3.48 (2H, triplet, J=8Hz)
$H_b$ 4.36 (2H, triplet, J=8Hz
$H_c$ 6.52 (1H, singlet).

Example 3

Determination of the antidotal activity for treatment of the soil. The test was carried out on maize of variety known as Decalbe XL 72 A, properly sowed into pots containing sandy soil.

The sowed pots were divided into three groups. One group was treated by spraying the soil with a composition containing the herbicide Eptam (in a quantity corresponding to 8 kg/ha of active substance), to

which the compound of formula (I) to be tested and used at the selected concentration was added; a second group (control) was treated with the herbicide only while the third group was treated neither with the herbicide nor with the antidote and was used as a check.

The pots were kept in a conditioned room (spring-growth conditions) at 15—24°C, 70% of relative humidity, 12-hour photoperiod, and were regularly sprinkled to secure a good germination.

10 days after treatment, the antidotal activity was evaluated by comparing the plant and the vegetative state thereof with the plants treated with the herbicide only and with those not treated at all.

The results were expressed on the basis of the plant's vegetative state according to a scale of values ranging from 4 (complete stop of the growth or death of the plant) to 0 (sound plant, growth like that of the check grown in the absence of both herbicide and antidote). Consequently a numerical evaluation like that of the plants treated with the herbicide only indicates the absence of the antidotal effect, and an evaluation equal to zero indicates full protection of the plant against the toxic action exerted by the herbicide; the intermediate values indicate a partial antidotal effect, increasing towards the lower values.

Preliminary laboratory tests indicated that the antidotes of formula (I) are not toxic towards maize, and that the herbicidal activity of Eptam towards the common infsting plants of Maize (Solanum nigrum, Amarantus spp., Echinochloa spp., Digitaria spp., Setaria spp., Sorghum halepense, Panichum dichotomiflorum, Cyperus rotundus and Cyperus esculentus) is not affected by the presence of the antidote in this kind of test.

The compounds of examples 1 and 2 (No. 1 and No. 2 respectively) exhibited a full antidotal activity (growth of the plant equal to the one of the check, evaluation = 0) at a dose of 400 g/ha (5% by weight in respect of the herbicide), while in the absence of the antidote, the herbicide Eptam, at the considered quantity caused a complete halt in the plants' development (evaluation = 4).

Example 4

Determination of the antidotal activity of Compound No. 2 at various doses for treatment of the soil in the defence of maize.

Following the same steps described in example 3, but using increasing quantities of the compounds being tested, the results recorded in the following Table and expressed as % of defence against the phytotoxic action of Eptam were obtained.

Table I

| Compounds | g/h | Quantities % referred to herbicide (Eptam = 8 kg/ha) | Activity defence in % |
|---|---|---|---|
| N° 2 (M 13302) | 24 | 0.3 | 100 |
| | 12 | 0.15 | 100 |
| | 6 | 0.075 | 100 |
| R-25788 (check compound) | 24 | 0.3 | 90 |
| | 12 | 0.15 | 50 |
| | 6 | 0.075 | 10 |

This table shows that M13302 (2-dichloromethyl-2-thiazoline) according to above Example 2 exert a much higher antidotal action than the R-25788 antidote of the prior art (N,N-dialkyldichloroacetamide) described in USP 4 021 224.

**Claims**

1. Method of defending useful plants against the phytotoxic effects of herbicides, consisting in treating the plants or parts thereof or the soil in which said plants grow with antidotally effective amounts, ranging from 0,03 to 10 parts by weight of herbicide, of a compound of general formula (I):

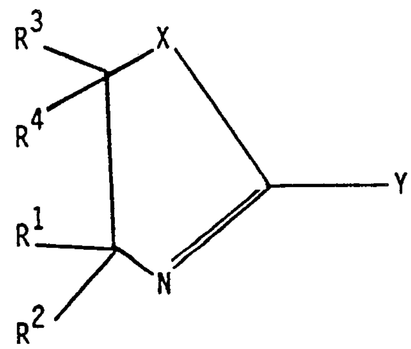

(I)

wherein:

X is an atom of S or of O;

Y is an alkyl $C_1$—$C_4$ substituted by one or more halogen atoms selected from the group consisting of Cl, Br and F;

$R^1$, $R^2$, $R^3$ and $R^4$, similar to or different from one another, are hydrogen or an alkyl $C_1$—$C_4$.

2. Useful compositions for reducing the damage caused by non-selective herbicides towards useful cultivations, containing one of the compounds of formula (I).

3. Compounds of general formula (I) wherein:

X is an atom of S;

Y is an alkyl $C_1$—$C_4$ substituted by one or more halogen atoms selected from the group consisting of Cl, Br, F provided that Y is not $CCl_3$;

$R^1$, $R^2$, $R^3$ and $R^4$, similar to or different from one another are hydrogen or an alkyl $C_1$—$C_4$.

4. The compounds according to claim 3 in which Y = $CHCl_2$.

5. The compounds according to claim 4 in which $R^1$, $R^2$, $R^3$ and $R^4$ are hydrogen.

**Patentansprüche**

1. Verfahren zum Schützen von Nutzplanzen gegen die phytotoxische Wirkung von Herbiziden, bei dem die Pflanzen oder Teile derselben oder der Boden, auf dem die Pflanzen wachsen, mit einer als Gegenmittel wirksamen Menge von 0,03 bis 10 Gewichtsteilen der toxischen Substanz einer der folgenden allgemeinen Formel (I) entsprechenden Verbindung behandelt werden:

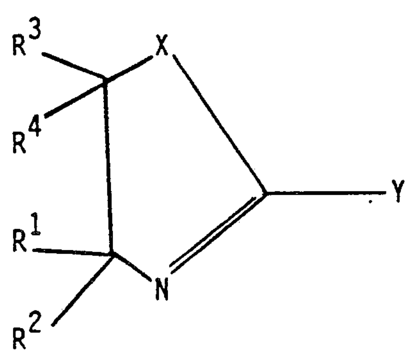

(I)

wobei:

X ein S-Atom oder ein O-Atom,

Y ein $C_1$—$C_4$ Alkyl, substituiert mit einem oder mehreren Halogenatomen der Gruppe Cl, Br und F

$R^1$, $R^2$, $R^3$ und $R^4$, die ähnlich oder verschieden sein können, Wasserstoff oder ein $C_1$—$C_4$ Alkyl darstellen.

2. Nützliche Präparate zur Verminderung der von nicht selektiven Herbiziden auf Nutzkulturen ausgeübten schädlichen Wirkungen, welche Präparate eine der Verbindungen gemäss vorstehender Formel (I) enthalten.

3. Verbindungen gemäss der allgemeinen Formel (I), bei welchen:

X ein S-Atom,

Y ein mit einem oder mehreren Halogenatomen der aus Cl, Br und F bestehenden Gruppe substituiertes, $C_1$—$C_4$ Alkyl ist, unter Ausschluss von C $Cl_3$, und

$R^1$, $R^2$, $R^3$ und $R^4$, die ähnlich oder verschieden sein können, Wasserstoff oder ein $C_1$—$C_4$ Alkyl darstellen.

4. Die Verbindungen nach Anspruch 3, bei welchen Y = CH $Cl_2$.
5. Die Verbindungen nach Anspruch 4, bei welchen $R^1$, $R^2$, $R^1$ und $R^4$ Wasserstoff sind.

**Revendications**

1. Procédé pour protéger des plantes utiles contre les effets phytotoxiques des herbicides, consistant à traiter les dites plantes ou des parties de celles-ci ou le sol sur lequel elles poussent avec des quantités efficaces du point de vue antidote, comprises entre 0,03 et 10 parties par rapport au poids de l'herbicide, d'un composé de formule générale (I):

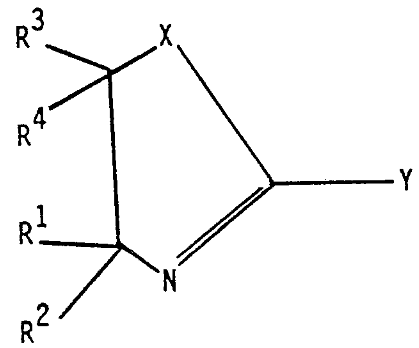

(I)

X représente un atome de S ou de O,
Y représente un radical alkyle en $C_1$ à $C_4$ substitué par un ou plusieurs atomes d'un halogène du groupe formé par $C_1$, Br et F,
$R^1$, $R^2$, $R^3$ et $R^4$, identiques ou différents, représentent de l'hydrogène ou un radical alkyle en $C_1$ à $C_4$.
2. Compositions utiles pour réduire les effets nocifs des herbicides non séléctifs sur les cultures utiles, lesquelles compositions renferment un des composés conformes à la formula (I).
3. Composés conformes à la formule générale (I) dans lesquels:
X représente un atome de S,
Y représente un radical alkyle en $C_1$ à $C_4$ substitué par 1 ou plusieurs atomes d'halogène choisi dans le groupe formé par Cl, Br et F, étant entendu que Y ne peut être C $Cl_3$,
$R^1$, $R^2$, $R^3$ et $R^4$, identiques ou différents, représentent de l'hydrogène ou un radical alkyle en $C_1$ à $C_4$.
4. Les composés selon la revendication 3, dans lesquels Y = CH $Cl_2$.
5. Les composés selon la revendication 4, dans lesquels $R^1$, $R^2$, $R^3$ et $R^4$ représentent de l'hydrogène.